Europäisches Patentamt

European Patent Office

Office européen des brevets

(19)

(11) **EP 0 984 831 B1**

(12) **EUROPÄISCHE PATENTSCHRIFT**

(45) Veröffentlichungstag und Bekanntmachung des Hinweises auf die Patenterteilung:
**08.08.2001** Patentblatt 2001/32

(21) Anmeldenummer: **98929266.9**

(22) Anmeldetag: **27.04.1998**

(51) Int Cl.$^7$: **B01J 25/00**

(86) Internationale Anmeldenummer:
**PCT/EP98/02481**

(87) Internationale Veröffentlichungsnummer:
**WO 98/53910 (03.12.1998 Gazette 1998/48)**

(54) **GEFORMTER, AKTIVIERTER METALL-FESTBETTKATALYSATOR**

MOULDED ACTIVATED METALLIC FIXED-BED CATALYST

CATALYSEUR METALLIQUE A LIT FIXE ACTIVE MOULE

(84) Benannte Vertragsstaaten:
**AT BE CH DE DK ES FR GB IE IT LI NL PT SE**

(30) Priorität: **26.05.1997 DE 19721898**

(43) Veröffentlichungstag der Anmeldung:
**15.03.2000** Patentblatt 2000/11

(73) Patentinhaber: **Degussa AG**
**40474 Düsseldorf (DE)**

(72) Erfinder:
• **FREUND, Andreas**
  **White Plains, NY 10606-2228 (US)**
• **BERWEILER, Monika**
  **D-63477 Maintal (DE)**
• **BENDER, Barbara**
  **D-63517 Rodenbach (DE)**
• **KEMPF, Bernd**
  **D-63839 Kleinwallstadt (DE)**

(56) Entgegenhaltungen:
**EP-A- 0 648 534**       **EP-A- 0 734 765**
**FR-A- 1 017 044**

**Beschreibung**

Die Erfindung betrifft einen geformten und in einer äußeren Schale aktivierten Metall-Festbettkatalysator nach Raney.

Aktivierte Metallkatalysatoren sind in der chemischen Technik als Raney-Katalysatoren bekannt. Sie werden überwiegend in Pulverform bei einer Größzahl von Hydrierungen, Dehydrierungen, Isomerisierungen und Hydratisierungen an organischen Verbindungen eingesetzt. Diese pulverförmigen Katalysatoren werden aus einer Legierung eines katalytisch aktiven Metalls, im folgenden auch als Katalysatormetall bezeichnet, mit einer in Alkalien löslichen weiteren Legierungskomponente hergestellt. Als Katalysatormetalle kommen hauptsächlich Nickel, Kobalt, Kupfer oder Eisen zum Einsatz. Für die in Alkalien lösliche Legierungskomponente wird vorwiegend Aluminium eingesetzt, aber auch andere Komponenten sind verwendbar, insbesondere sind auch Zink und Silizium oder deren Mischungen mit Aluminium geeignet.

Die Herstellung dieser sogenannten Raney-Legierungen erfolgt überwiegend nach dem Barrengußverfahren. Dabei wird zunächst eine Mischung aus dem Katalysatormetall und zum Beispiel Aluminium geschmolzen und dann zu Barren vergossen. Typische Legierungsansätze im Produktionsmaßstab belaufen sich auf etwa 10 bis 100 kg pro Barren. Gemäß der DE-OS 21 59 736 liegen die hierbei erzielbaren Abkühlzeiten innerhalb von 2 Stunden. Dies entspricht einer mittleren Abkühlgeschwindigkeit von cirka 0,2 K/s. Im Gegensatz dazu werden bei Verfahren zur raschen Abkühlung (zum Beispiel Verdüsungsverfahren) Abkühlgeschwindigkeiten von $10^2$ bis $10^6$ K/s erreicht. Die Abkühlgeschwindigkeit wird insbesondere durch die Partikelgröße und das Abkühlmedium beeinflußt (siehe: Materials Science and Technology; Eds. R.W.Chan, P.Haasen, E.J.Kramer; Vol 15 [Processing of Metals and Alloys] 1991, VCH-Verlag Weinheim, Seiten 57 bis 110). Ein derartiges Verfahren wird in EP 0 437 788 B1 angewendet, um ein Raney-Legierungspulver herzustellen. Hierbei wird die Schmelze der Legierung mit einer Temperatur von 50 bis 500°C über ihrem Schmelzpunkt mit Wasser und/oder einem Gas verdüst und abgekühlt.

Zur Herstellung eines Katalysators wird die Raney-Legierung zunächst, sofern sie nicht schon bei der Herstellung in der gewünschten Pulverform anfällt, fein vermahlen. Anschließend wird das Aluminium durch Auslaugen mit Alkalien, wie zum Beispiel Natronlauge, ganz oder teilweise entfernt. Damit wird das Legierungspulver aktiviert. Durch Auslaugen des Aluminiums weist das Legierungspulver eine hohe spezifische Oberfläche zwischen 20 und 100 $m^2$/g auf und ist reich an adsorbiertem Wasserstoff. Das aktivierte Katalysatorpulver ist pyrophor und wird unter Wasser, organischen Lösungsmitteln oder durch Einbetten in bei Raumtemperatur feste, organische Verbindungen gelagert.

Pulverkatalysatoren haben den Nachteil, daß sie nur in Batch-Verfahren eingesetzt werden können und nach der katalytischen Umsetzung durch aufwendige Sedimentation und/oder Filtration von den Reaktionsmedien abgetrennt werden müssen. Es sind daher verschiedene Verfahren zur Herstellung von Formkörpern bekannt geworden, die nach Auslaugen des Aluminiums zu aktivierten Metall-Festbettkatalysatoren führen. So sind zum Beispiel grobstückige, d.h. nur grob vermahlene Raney-Legierungen erhältlich, die durch Behandeln mit Natronlauge aktiviert werden können. Das Auslaugen und Aktivieren findet dabei nur in einer oberfächlichen Schale statt, deren Dicke durch die Auslaugbedingungen einstellbar ist.

Ein wesentlicher Nachteil der nach diesem Verfahren hergestellen Katalysatoren ist die schlechte mechanische Stabilität der aktivierten Außenschicht. Da nur diese Außenschicht des Katalysators auch katalytisch aktiv ist, führt Abrieb zu einer schnellen Desaktivierung, die dann bestenfalls durch eine erneute Aktivierung von tieferliegenden Legierungsschichten mit Natronlauge teilweise rückgängig gemacht werden kann.

In der Patentanmeldung EP 0 648 534 A1 werden geformte, aktivierte Metall-Festbettkatalysatoren nach Raney und deren Herstellung beschrieben, die die zuvor geschilderten Nachteile, wie z.B. die schlechte mechanische Stabilität bei einer schalenförmigen Aktivierung vermeiden. Zur Herstellung dieser Katalysatoren wird eine Mischung aus Pulvern einer Katalysatorlegierung und eines Bindemittels verwendet, wobei die Katalysatorlegierungen jeweils mindestens ein katalytisch aktives Katalysatormetall und eine auslaugbare Legierungskomponente enthalten. Als Bindemittel werden die reinen Katalysatormetalle oder Mischungen davon verwendet, die keine auslaugbare Komponente enthalten. Der Einsatz des Bindemittels in einer Menge von 0,5 - 20 Gew.-%, bezogen auf die Katalysatorlegierung, ist wesentlich, um eine ausreichende mechanische Stabilität nach der Aktivierung zu erreichen. Nach der Verformung der Katalysatorlegierung und des Bindemittels mit üblichen Verformungshilfsmitteln und Porenbildnern werden die erhaltenen Grünkörper bei Temperaturen von unter 850°C kalziniert. Hierbei kommt es durch Sinterprozesse des feinteiligen Bindemittels zur Ausbildung von festen Verbindungen zwischen den einzelnen Körnern der Katalysatorlegierung. Diese Verbindungen sind im Gegensatz zu den Katalysatorlegierungen nicht oder nur in geringerem Maße laugbar, sodaß auch nach der Aktivierung eine mechanisch stabile Struktur erhalten bleibt. Das zugesetzte Bindemittel hat jedoch den Nachteil, daß es im wesentlichen katalytisch inaktiv ist und somit die Zahl der aktiven Zentren in der aktivierten Schale verringert. Darüber hinaus erlaubt der zwingend notwendige Einsatz des Bindemittels nur eine beschränkte Variationsbreite der Menge an Porenbildner, ohne die Festigkeit des Formkörpers zu gefährden. Aus diesem Grunde ist auch die Schüttdichte dieser Katalysatoren ohne Verlust an Festigkeit kaum unter einen Wert von 1,9 Kg/l zu vermindern. Dies bedeutet einen erheblichen wirtschaftlichen Nachteil beim Einsatz dieser Katalysatoren in technischen Prozessen. Insbesondere bei Verwendung teurer Katalysatorlegierungen, zum Beispiel Kobaltlegierungen, führt die hohe Schüttdichte zu einer hohen Kapitalbindung pro Reaktorfüllung, die jedoch teilweise durch die hohe Aktivität und Langzeitstabilität dieser Katalysatoren ausgeglichen wird. In bestimmten Fällen kann die hohe Schüttdichte der Katalysatoren auch eine mechanisch verstärkte Reaktorkonstruktion erfordern.

Aufgabe der vorliegenden Erfindung ist es daher, einen geformten, aktivierten Metall-Festbettkatalysator anzugeben, der die angeführten Nachteile der bekannten Festbettkatalysatoren weitgehend vermeidet.

Diese Aufgabe wird gelöst durch einen geformten, aktivierten Metall-Festbettkatalysator mit einem Porenvolumen von 0,05 bis 1 ml/g und einer äußeren, aktivierten Schale, bestehend aus einer versinterten, feinteiligen Katalysatorlegierung sowie gegebenenfalls Promotoren, wobei die Katalysatorlegierung aus der Herstellung der Legierung resultierende metallurgische Phasendomänen aufweist, deren volumenmäßig größte Phase eine spezifische Grenzflächendichte von mehr als 0,5 $\mu m^{-1}$ besitzt.

Bei der spezifischen Grenzflächendichte $S_v$ handelt es sich um eine metallografische Kenngröße, die die Feinkörnigkeit des Phasengefüges einer Legierung beschreibt und durch folgende Beziehung definiert ist:

$$S_v = \frac{4}{\pi} \cdot \frac{\text{Umfang(Phase)}}{\text{Fläche(Phase)}} \ [\mu m^{-1}]$$

Diese Kenngröße wird zum Beispiel in der US 3,337,334 als "Komplexitätsindex" C.I. eingeführt. Die hier definierte spezifische Grenzflächendichte $S_v$ unterscheidet sich von dem in der US 3,337,334 angegebenen Komplexizitätsindex nur durch die Proportionalitätskonstante $4/\pi$. Je größer $S_v$ ist, um so kleiner sind die entsprechenden Phasendomänen.

Wesentlich für den erfindungsgemäßen Katalysator ist ein möglichst kleinvolumiges Phasengefüge. Ein solches Phasengefüge ist gegeben, wenn die Phase mit dem größten Volumenanteil in der Legierung eine spezifische Grenzflächendichte von mehr als 0,5 $\mu m^{-1}$ aufweist.

Die spezifische Grenzflächendichte kann gemäß der US 3,337,334 durch eine quantitative, metallografische Untersuchung ermittelt werden. Hierzu wird ein Querschliff durch ein Korn der Katalysatorlegierung angefertigt und mikroskopisch untersucht. Die unterschiedlichen Phasen der Katalysator-Legierungen zeigen im Lichtmikroskop bereits im polierten, insbesondere im kontrastierten oder geätzten Zustand unterschiedliche Grautöne. Anhand der unterschiedlichen Grauwerte können die Gefüge mit Hilfe eines automatischen PC-gestützten Bildanalyse-Systems ausgewertet werden. Die vorliegenden Phasen können durch Analyse der Zusammensetzung mittels energiedispersiver Röntgenanalyse identifiziert werden. Zum Beispiel werden für eine Nickel/Aluminium -Legierung mit der Zusammensetzung von etwa 50 Gew.-% Nikkel und 50 Gew.-% Aluminium entsprechend dem Phasendiagramm (siehe ASM Specialty Handbook® "Aluminum and Aluminum Alloys"; Eds. J.R. Davis, 3. Auflage, S. 522, 1994) die Phasen $Al_3Ni_2$, $Al_3Ni$ sowie ein $Al$-$Al_3Ni$-Eutektium beobachtet.

Zur Charakterisierung der Katalysatorlegierung wird zunächst die Phase mit dem größten Volumenanteil an der Legierung bestimmt. Die spezifische Grenzflächendichte wird dann zweckmäßiger Weise mit Hilfe moderner Bildanalyseverfahren für diese Phase ermittelt.

Es wurde überraschenderweise gefunden, daß bei Einsatz von Katalysatorlegierungen mit einem kleinvolumigen Phasengefüge, dessen volumenmäßig größte Phase eine spezifische Grenzflächendichte von größer als 0,5 $\mu m^{-1}$ aufweist, die erfindungsgemäßen Katalysatoren ohne Zusatz eines Bindemittels hergestellt werden können. Trotz fehlendem Bindemittel wird eine mechanisch stabile Struktur mit hoher Abriebsfestigkeit ausgebildet. Bei der Herstellung des Katalysators kann Porenbildner wegen des fehlenden Bindemittels in größeren Mengen zugesetzt werden als bei dem aus dem Stand der Technik bekannten Katalysator. Dies ermöglicht die Herstellung von Katalysatorkörpern mit einem größeren Porenvolumen. Der fehlende, weitgehend inerte Binder, und das höhere Porenvolumen führen zu Katalysatoren mit hoher volumenspezifischer Aktivität.

Katalysatorlegierungen nach Raney werden typischerweise aus der Schmelze des Katalysatormetalls und Aluminium gewonnen. Je nach Art und Geschwindigkeit der Abkühlung der Schmelze können sich trotz identischer, makroskopischer Zusammensetzung unterschiedliche Phasengefüge ergeben. Beim Gießen von Barren bildet sich in der Regel wegen der geringen Abkühlgeschwindigkeiten ein grobes Phasengefüge mit großflächigen Phasendomänen aus. Wird dagegen für eine raschere Abkühlung gesorgt, wird ein wesentlich feineres Gefüge erzeugt. Die notwendigen Abkühlgeschwindigkeiten kann der Fachmann leicht durch entsprechende Untersuchungen festlegen. Beim Barrengußverfahren können nur noch kleine Legierungsansätze verarbeitet werden. Als Richtwert für die erforderlichen Abkühlgeschwindigkeiten können Abkühlzeiten von der Schmelztemperatur auf bis unter 700°C von weniger als 2 Minuten genannt werden. Dies entspricht Abkühlgeschwindigkeiten von wenigstens 5 K/s. Bevorzugt werden Abkühlgeschwindigkeiten von mehr als 10, insbesondere von mehr als 50 K/s angewendet. Das Pulverherstellungsverfahren gemäß der EP 0 437 788 B1 liefert im allgemeinen Legierungspulver mit einem geeigneten Phasengefüge.

Für die Herstellung des erfindungsgemäßen Katalysators können Legierungspulver mit mittleren Partikelgrößen von 10 $\mu m$ bis 1000 $\mu m$ eingesetzt werden. Daneben ist für hochaktive Katalysatoren auch die Schüttdichte des Legierungspulvers von Bedeutung. Sie sollte in einem Bereich zwischen 1,0 und 3,0 Kg/l liegen. Bei Schüttdichten über 3,0 Kg/l werden zu kompakte und damit wenig aktive Katalysatoren erhalten. Schüttdichten von weniger als 1,0 Kg/l führen zu mangelhafter mechanischer Stabilität der Katalysatorkörper. Eine besonders hohe Volumenaktivität der Katalysatoren kann erzielt werden, wenn das Legierungspulver eine mittlere Korngröße von größer als 100 µm und ein Schüttgewicht von kleiner als 1,6 kg/l aufweist.

Das Gewichtsverhältnis zwischen Katalysatormetall und der auslaugbaren Legierungskomponente in der Katalysatorlegierung liegt, wie bei Raney-Legierungen üblich, im Bereich zwischen 20 : 80 und 80 : 20. Die erfindungsgemäßen Katalysatoren können zur Beeinflussung der katalytischen Eigenschaften noch mit anderen Metallen dotiert sein. Ziel einer solchen Dotierung ist zum Beispiel die Verbesserung der Selektivität in einer bestimmten Reaktion. Die Dotierungsmetalle werden häufig auch als Promotoren bezeichnet. Dotierung bzw. Promotierung von Raney-Katalysatoren werden zum Beispiel in der US 4,153.578, in der DE-AS 21 01 856, in der DE-OS 21 00 373 und in der DE-AS 20 53 799 beschrieben.

Prinzipiell können alle bekannten Metallegierungen mit laugbaren Elementen wie Aluminium, Zink und Silizium für die vorliegende Erfindung eingesetzt werden. Geeignete Promotoren sind die Übergangsmetalle der Gruppen IIIb bis VIIb und VIII und der Gruppe IB des periodischen Systems der Elemente sowie die Seltenerdmetalle. Sie werden in einer Menge von bis zu 20 Gew.-%, bezogen auf das Gesamtgewicht des Katalysators, eingesetzt. Vorzugsweise werden Chrom, Mangan, Eisen, Kobalt, Vanadin, Tantal, Titan, Wolfram und/oder Molybdän sowie die Metalle der Platingruppe als Promotoren verwendet. Zweckmäßigerweise werden sie schon als Legierungsbestandteil der Katalysatorlegierung zugefügt. Darüber hinaus können Promotoren mit einer anderen laugbaren Metalllegierung, in Form eines separaten Metallpulvers oder nachträglich auf die Katalysatorkörper aufgebracht werden. Die nachträgliche Aufbringung von Promotoren kann sowohl nach der Kalzinierung als auch nach der Aktivierung erfolgen. Dadurch ist eine optimale Abstimmung der katalytischen Eigenschaften auf den jeweiligen katalytischen Prozeß möglich.

Katalysatorlegierung und gegebenenfalls Promotoren werden in Form von Pulvern unter Zusatz von Befeuchtungsmitteln und/oder Zuschlagstoffen wie Verformungshilfsmitteln, Gleitmitteln, Plastifizierern und gegebenenfalls Porenbildnern zu einer verformbaren Masse verarbeitet. Als Gleitmittel, Plastifizierer und Porenbildner können alle hierfür üblichen Materialien eingesetzt werden. In den US-Patentschriften US 4,826,799, US 3,404,551 und US 3,351,495 wird eine Vielzahl geeigneter Materialien genannt. Bevorzugt werden Wachse, wie zum Beispiel Wachs C Mikropulver PM der Hoechst AG, Fette wie Magnesium- oder Aluminiumstearate oder Kohlehydrate enthaltende Polymere wie Tylose (Methylcellulose) eingesetzt. Die Feststoffe der Mischung werden, falls notwendig unter Zugabe eines Befeuchtungsmittels, sorgfältig in geeigneten Mischern oder Knetern homogenisiert. Als Befeuchtungsmittel sind Wasser, Alkohole, Glykole, Polyetherglykole oder deren Mischungen geeignet. Ziel dieser Homogenisierung ist die Vorbereitung der Mischung für das nachfolgende Formgebungsverfahren. Anwendbar sind beispielsweise die Extrusion, Tablettierung und Kompaktierung. Art und Reihenfolge der Einbringung der Zuschlagstoffe hängen von dem anzuwendenden Formgebungsverfahren ab. So erfordert die Extrusion eine plastische Masse bestimmter Viskosität, während für die Tablettierung ein rieselfähiges und leicht dosierbares Material notwendig ist. Die hierfür anzuwendenden Techniken wie zum Beispiel eine Agglomeration zur Bildung eines rieselfähigen Pulvers oder die Einstellung der richtigen Viskosität für die Extrusion werden vom Fachmann routinemäßig eingesetzt.

Als Formkörper kommen alle in der Katalysatortechnik üblichen Formen in Betracht. Es können beispielsweise je nach Erfordernissen des Anwendungsfalles Extrudate, Kugeln, Ringe, Speichenringe oder Tabletten hergestellt werden. Die fertigen Formkörper werden soweit erforderlich bis zur Gewichtskonstanz bei Temperaturen zwischen 80 und 120°C getrocknet und anschließend bei Temperaturen unterhalb von 850°C, bevorzugt zwischen 200 und 700°C, an Luft in kontinuierlich oder diskontinuierlich arbeitenden Öfen wie Drehrohröfen, Bandkalzinierer oder stationären Öfen kalziniert. Dabei verbrennen die organischen Zuschlagstoffe und lassen ein entsprechendes Porensystem zurück.

Porenstruktur und Porenvolumen der Katalysatoren können durch geeignete Wahl und Menge der porenbildenden Zuschlagstoffe in weiten Bereichen variiert werden. Die endgültig sich ausbildende Porenstruktur und das Porenvolumen werden auch durch die mittlere Partikelgröße des eingesetzten Katalysatorlegierungspulvers und die Art der Kompaktierung beeinflußt. Durch entsprechende Wahl der genannten Parameter kann die Struktur der Formkörper an die Erfordernisse des jeweiligen katalytischen Prozesses angepaßt werden.

Beim Kalzinieren der Formkörper sintern die Partikel des Legierungspulvers zusammen und verleihen den Formkörpern eine hohe mechanische Stabilität und gute Abriebfestigkeit. Typischerweise liegt die Härte von zylinderförmigen Tabletten nach dem Kalzinieren bei Werten zwischen 50 und 400 N (radial gemessen nach ASTM D 4179-82). Durch die hohe, spezifische Grenzflächendichte und die damit erhöhte Reaktivität für Festkörperreaktionen kommt es unter den gewählten Kalzinationsbedingungen zur Ausbildung einer stabilen, porösen Struktur. Beim Vergleich der Phasengefüge vor und nach der Kalzination fällt auf, daß die gewünschte Festkörperreaktion nur mit einer geringfügigen Verringerung der spezifischen Grenzflächendichte einhergeht. Die spezifische Grenzflächendichte liegt jedoch auch bei den fertigen Katalysatorformkörpern noch oberhalb von 0,5 µm$^{-1}$.

Für die wirtschaftliche Nutzung der Erfindung sind die nach dem Kalzinieren resultierenden Katalysatorvorstufen ebenfalls von großer Bedeutung. Sie sind noch nicht pyrophor und können daher ohne Schwierigkeiten gehandhabt und transportiert werden. Die Aktivierung kann dann vom Anwender kurz vor Gebrauch vorgenommen werden. Eine Lagerung unter Wasser oder organischen Lösungsmitteln oder die Einbettung in organischen Verbindungen ist für die Katalysatorvorstufen nicht erforderlich.

Gegenüber den fertigen Katalysatoren handelt es sich bei den Katalysatorvorstufen um Körper mit homogener Zusammensetzung aus einer innigen Mischung von Partikeln der Katalysatorlegierung und gegebenenfalls einem oder mehreren Promotoren, die zu einem mechanisch stabilen und porösen Formkörper versintert sind. Seine Dichte liegt je nach Zusammensetzung der Katalysatorlegierung und abhängig vom Porenvolumen zwischen 1,0 und 2,5 Kg/l. Vorteilhaft sind Porenvolumina zwischen 0,05 bis 1 ml/g. Da die Katalysatorvorstufen noch nicht aktiviert sind, beträgt ihre spezifische Oberfläche weniger als 20, in der Regel weniger als 10 m$^2$/g.

Die Katalysatorvorstufe besteht zu mehr als 99 Gew.-% aus der Katalysatorlegierung und den gegebenenfalls enthaltenen Promotoren. Beim Kalzinieren der Vorstufe bei Temperaturen unter 850°C können sich geringfügige Anteile von oberflächlichen Oxiden bilden, die jedoch bei der Laugenaktivierung entfernt werden und daher auf die späteren katalytischen Eigenschaften keinen Einfluß haben.

Nach dem Kalzinieren werden die Formkörper durch Auslaugen des Aluminiums mit Hilfe von Natronlauge aktiviert. Hierzu kann z.B. eine auf 80°C erwärmte 20%-ige Natronlauge verwendet werden. Eine Behandlungsdauer von 2 Stunden führt dabei je nach Porosität des kalzinierten Formkörpers zu einer aktiven Schale von etwa 0,1 bis 1,0 mm Dicke. Insbesondere hat sich dabei gezeigt, daß sich die Härte der mit geringerem Druck kompaktierten Formkörper durch das Auslaugen deutlich erhöht.

Die nachfolgenden Beispiele dienen dem weiteren Verständnis der Erfindung. Obwohl in den Beispielen nur einige bevorzugte Ausführungsformen der Erfindung vorgestellt werden, erlaubt die vorliegende Erfindung dem Fachmann, aktivierte Metall-Festbettkatalysatoren nach Raney mit in weiten Bereichen variierenden Parametern herzustellen und sie den jeweiligen Anwendungserfordernissen anzupassen.

Als Maßzahl für die Menge der katalytisch aktiven Zentren der Katalysatoren wurde in einigen Beispielen ihre Sauerstoffaufnahme mittels temperaturprogrammierter Oxidation (TPO) ermittelt. Im Falle von Nickelkatalysatoren kann zum Beispiel jedes aktivierte Nickelatom durch Oxidation ein Sauerstoffatom aufnehmen.

Zur Durchführung der TPO wurden ca. 5 bis 10 g der wasserfeuchten, aktivierten Katlysatorkörper in einem U-förmigen Quarzglasrohr (Innendurchmesser: 1cm, Schenkellänge: 15 cm) im Stickstoffstrom von 10 1/h bei 120°C für die Dauer von 17 Stunden getrocknet. Der Ofen wurde danach mit flüssigem Stickstoff vorsichtig auf -190°C abgekühlt. Nach Erreichen einer konstanten Reaktortemperatur wurde der Stickstoffstrom abgestellt und ein 4 Vol.-% Sauerstoff enthaltendes Stickstoffgas mit 10 l/h über die Probe geleitet. Die Analyse des Sauerstoffgehalts der Probe erfolgte mit einem "Oxynos 100" der Firma Leybold-Heraeus, das auf einem paramagnetischen Meßprinzip beruht. Nach Erreichen eines konstanten Sauerstoffgehalts bei ca. -120°C wurde eine Temperaturrampe von 6°C/min angelegt. Das Profil der Sauerstoffaufnahme wurde im Temperaturbereich von -100°C bis +550°C erfaßt. Die Menge des aufgenommenen Sauerstoffs wurde aus der Fläche unter dem Profil der Sauerstoffaufnahme ermittelt. Die Menge des aufgenommen Sauerstoffs wird in mmol $O_2$/g Katalysator angegeben.

Für die Herstellung von erfindungsgemäßen Katalysatoren wurden die in Tabelle 1 aufgeführten Metallegierungen A bis E verwendet. Diese Legierungspulver wurden bei ihrer Herstellung aus einer Schmelze besonders schnell abgekühlt und weisen daher ein sehr kleinvolumiges Phasengefüge auf. Bei dem zur Herstellung eines Vergleichskatalysators verwendeten Legierungspulver V handelt es sich um ein Material, welches standardmäßig für die Herstellung von aktivierten Metall-Pulverkatalysatoren und Metall-Festbettkatalysatoren verwendet wird.

Die Bestimmung der spezifischen Grenzflächendichte $S_v$ ist sehr aufwendig und wurde daher nur an den Legierungspulvern V und A und an einer Referenzprobe exakt ermittelt. Die Grenzflächendichte der Legierungspulver B bis E wurden qualitativ durch Vergleich mit der Referenzprobe bestimmt. Für die Herstellung der Referenzprobe wurden 100 g grobe Bruchstücke der NiAl-Legierung V (Tabelle 1) aufgeschmolzen und 4 Stäbe mit einem Durchmesser von 7 mm und einer Länge von 15 cm gegossen. Durch die große Oberfläche der Stäbe konnte eine höhere Abkühlgeschwindigkeit erreicht werden. Die Abkühlzeit dieser Stäbe von Schmelztemperatur auf unter 700°C betrug etwa 1 Minute.

Mit der schon beschriebenen quantitativen Metallografie wurde für die $Ni_2Al_3$-Phase dieser Referenzprobe ein Volumenanteil von 67,4% und eine spezifische Grenzflächendichte von 0,5 $\mu m^{-1}$ gemessen. Zur Abschätzung der spezifischen Grenzflächendichten der Legierungen B bis E wurden lichtmikroskopische Aufnahmen von Querschliffen bei 200 und 500-facher Vergrößerung angefertigt und mit entsprechenden Aufnahmen der Referenzprobe verglichen.

Tabelle 1:

| Legierungspulver | | | |
|---|---|---|---|
| **Legierung** | **Bezeichnung** | | **Daten** |
| V | Ni/Al-Verhältnis | | 53/47 |
| | $S_v$ (Ni$_2$Al$_3$ 65.7 Vol.-%) | [µm$^{-1}$] | 0,08 |
| | volumenbezogener, mittlerer Korndurchmesser | [µm] | 50 |
| | Schüttgewicht | [Kg/l] | 2,23 |
| A | Ni/Al-Verhältnis | | 50/50 |
| | $S_v$ (Ni$_2$Al$_3$ 57,1 Vol.-%) | [µm$^{-1}$] | 1,6 |
| | volumenbezogener, mittlerer Korndurchmesser | [µm] | 124 |
| | Schüttgewicht | [Kg/l] | 1,40 |
| B | Ni/Al-Verhältnis | | 50/50 |
| | $S_v$ | [µm$^{-1}$] | >0,5 |
| | Kornfraktion | [µm] | 500-1400 |
| | Schüttgewicht | [Kg/l] | 1,6 |
| C | Ni/Cr/Al-Verhältnis | | 50/1,5/48,5 |
| | $S_v$ | [µm$^{-1}$] | >0,5 |
| | volumenbezogener, mittlerer Korndurchmesser | [µm] | 32 |
| | Schüttgewicht | [Kg/l] | 1,47 |
| D | Cu/Al-Verhältnis | | 50/50 |
| | $S_v$ | [µm$^{-1}$] | >0,5 |
| | volumenbezogener, mittlerer Korndurchmesser | [µm] | 48 |
| | Schüttgewicht | [Kg/l] | 1,48 |
| E | Cu/Zn/Al-Verhältnis | | 50/15/35 |
| | $S_v$ | [µm$^{-1}$] | >0,5 |
| | volumenbezogener, mittlerer Korndurchmesser | [µm] | 50 |
| | Schüttgewicht | [Kg/l] | 1,75 |

**Vergleichsbeispiel 1:**

Gemäß der Vorschrift der EP 0 648 534 A1 wurde für einen Vergleichskatalysator aus 1000 g Legierungspulver V, 150 g Ni-Pulver (> 99% Ni; $d_{50}$ = 21 µm; 15 Gew.-% bezogen auf das eingesetzte Legierungspulver) und 25 g Ethylenbisstearoylamid (2.13 Gew.-% bezogen auf den gesamten Metallgehalt) unter Zugabe von ca. 150 g Wasser eine leicht rieselfähige, tablettierbare Katalysatormischung hergestellt. Aus dieser wurden Tabletten mit einem Durchmesser von 4 mm und einer Höhe von 4 mm gepreßt. Die Kalzination der Formkörper wurde 2 h bei 700°C durchgeführt. Danach betrug das mittlere Gewicht einer Tablette 193,4 mg. Die Tabletten wurden nach dem Kalzinieren in 20%-iger Natronlauge 2 h bei 80°C aktiviert. Die mittlere Dicke der aktivierten Schale, bestimmt mit einem Lichtmikroskop, lag bei 0,2 mm. Die Sauerstoffaufnahme des fertigen Katalysators als Maß für die Zahl der aktiven Nickelzentren wurde mittels TPO bestimmt und lag bei 1,04 mmol O$_2$/g Katalysator.

**Beispiel 1:**

Aus 1000 g Legierungspulver A und 21,3 g Ethylenbisstearoylamid (2.13 Gew.-% bezogen auf den gesamten Metallgehalt) wurde eine leicht rieselfähige, tablettierbare Katalysatormischung hergestellt. Aus dieser Mischung wurden analog dem Vergleichsbeispiel 1 aktivierte Tabletten hergestellt. Das mittlere Gewicht einer Tablette nach der Kalzination betrug nur 157,3 mg. Bei gleicher Aktivierungsdauer betrug die mittlere Dicke der aktivierten Schale 0,47 mm. Die Sauerstoffaufnahme des fertigen Katalysators lag bei 2,16 mmol $O_2$/g Katalysator.

**Beispiel 2:**

Aus 1000 g Legierungspulver B und 21,3 g Ethylenbisstearoylamid (2,13 Gew.-% bezogen auf den gesamten Metallgehalt) wurde eine leicht rieselfähige, tablettierbare Katalysatormischung hergestellt. Aus dieser Mischung wurden analog dem Vergleichsbeispiel 1 aktivierte Tabletten hergestellt. Das mittlere Gewicht einer Tablette nach Kalzination betrug 185,8 mg. Bei gleicher Aktivierungsdauer betrug die mittlere Dicke der aktivierten Schale 0,65 mm.

Mit der gleichen Kornfraktion des Legierungspulvers A konnten trotz Zusatz von Bindemittel keine stabilen Formkörper nach der Aktivierung erhalten werden.

**Beispiel 3:**

Aus 1000 g Legierungspulver C und 21,3 g Ethylenbisstearoylamid (2.13 Gew.-% bezogen auf den gesamten Metallgehalt) wurde eine leicht rieselfähige, tablettierbare Katalysatormischung hergestellt. Aus dieser Mischung wurden analog dem Vergleichsbeispiel 1 aktivierte Tabletten hergestellt. Das mittlere Gewicht einer Tablette betrug nur 152,1 mg. Bei gleicher Aktivierungsdauer betrug die mittlere Dicke der aktivierten Schale 0,3 mm.

**Vergleichsbeispiel 2:**

Für einen Vergleichskatalysator gemäß der EP 0 648 534 A1 wurden aus 1000 g Legierungspulver C, 150 g Ni-Pulver (> 99% Ni; $d_{50}$ = 21 μm; 15 Gew.-% bezogen auf das eingesetzte Legierungspulver) und 25 g Ethylenbisstearoylamid (2,13 Gew.-% bezogen auf den gesamten Metallgehalt) eine leicht rieselfähige, tablettierbare Katalysatormischung hergestellt. Aus dieser Mischung wurden analog dem Vergleichsbeispiel 1 aktivierte Tabletten hergestellt. Das mittlere Gewicht einer Tablette betrug 167,7 mg. Bei gleicher Aktivierungsdauer betrug die mittlere Dicke der aktivierten Schale 0,3 mm.

**Beispiel 4:**

Aus 1000 g Legierungspulver D und 43 g Ethylenbisstearoylamid (4,3 Gew.-% bezogen auf den gesamten Metallgehalt) wurde eine leicht rieselfähige, tablettierbare Katalysatormischung hergestellt. Aus dieser Mischung wurden analog dem Vergleichsbeispiel 1 aktivierte Tabletten hergestellt. Das mittlere Gewicht einer Tablette nach der Kalzination betrug nur 168,9 mg. Bei gleicher Aktivierungsdauer betrug die mittlere Dicke der aktivierten Schale 0,3 mm.

Aus einem Legierungspulver mit identischer, makroskopischer Zusammensetzung, jedoch mit einer spezifischen Grenzflächendichte von kleiner 0,5 μm$^{-1}$, konnten mit derselben Menge Ethylenbisstearoylamid keine stabilen Formkörper nach der Aktivierung erhalten werden.

**Beispiel 5:**

Aus 1000 g Legierungspulver E und 43 g Ethylenbisstearoylamid (4,3 Gew.-% bezogen auf den gesamten Metallgehalt) wurde eine leicht rieselfähige, tablettierbare Katalysatormischung hergestellt. Aus dieser Mischung wurden analog dem Vergleichsbeispiel 1 aktivierte Tabletten hergestellt. Das mittlere Gewicht einer Tablette nach der Kalzination betrug nur 186,5 mg. Bei gleicher Aktivierungsdauer betrug die Dicke der aktivierten Schale 0,35 mm.

**Anwendungsbeispiel 1:**

Die Katalysatoren der Vergleichsbeispiele 1 und 2 sowie der Beispiele 1 bis 3 wurden bezüglich ihrer katalytischen Aktivität bei der Hydrierung von Nitrobenzol miteinander verglichen. Dazu wurden 100 g Nitrobenzol und 100 g Ethanol in einen Rührautoklaven mit Begasungsrührer von 0,5 l Fassungsvermögen eingefüllt. In den Rührautokaven wurden jeweils 10 g des zu untersuchenden Katalysators mittels eines Katalysatorkorbes so eingehängt, daß die Katalysatorformkörper gut vom Edukt/Lösungsmittel-Gemisch umspült und mit Wasserstoff begast wurden. Die Hydrierung wurde bei einem Wasserstoffdruck von 40 bar und einer Temperatur von 150°C durchgeführt. Es wurde die Anspringtemperatur sowie die Geschwindigkeit der Wasserstoffaufnahme ermittelt. Die Ergebnisse sind in Tabelle 2 aufgelistet. Zur Kontrolle wurden jeweils nach 1, 2, 3, 4 und 5 Stunden Reaktionszeit Proben gezogen und mittels eines Gaschromatografen analysiert.

Tabelle 2:

| Hydrierung von Nitrobenzol zu Anilin | | |
|---|---|---|
| Beispiel | Anspringtemperatur [°C] | Wasserstoffaufnahmegeschwindigkeit [l/h·g$_{Kat.}$] |
| VB 1 | 131 | 1,14 |
| B 1 | 115 | 1,59 |
| B 2 | 85 | 2,29 |
| B 3 | 112 | 2,12 |
| VB 2 | 118 | 1,88 |

Die Ergebnisse von Tabelle 2 zeigen, daß die erfindungsgemäßen Katalysatoren B 1 und B2 bzw. B 3 gegenüber den Vergleichskatalysatoren VB 1 bzw. VB 2 eine wesentlich gesteigerte Aktivität bei der Hydrierung von Nitrobenzol zu Anilin aufweisen. Die geringere Aktivität der Vergleichskatalysatoren kann auf den geringeren Gehalt an aktiviertem Metall bedingt durch den Einsatz des weitgehend inerten Binders zurückgeführt werden.

**Anwendungsbeispiel 2:**

In einem Rohrreaktor (d = 25,4 mm, l = 295 mm) wurde mit 25 ml des Katalysators aus Vergleichsbeispiel 1 bzw. Beispiel 1 in der Rieselphase Aceton zu Isopropanol hydriert. Die Hydrierung wurde anfänglich bei 5 bar Wasserstoffdruck, 70°C und einer LHSV von 0,2 h$^{-1}$ durchgeführt. Im Verlauf der Untersuchung wurde die LHSV schrittweise um 0,2 auf 1,2 h$^{-1}$ angehoben. Die Temperatur erhöhte sich durch die Exothermie auf ca. 80°C. Nach 20 Stunden Laufzeit wurde eine Probe des Produkts genommen und hinsichtlich Umsatz und Isopropanolselektivität untersucht (Tabelle 3):

Tabelle 3:

| Hydrierung von Aceton zu Isopropanol | | |
|---|---|---|
| Beispiel | Umsatz [%] | Selektivität bzgl. Isopropanol [%] |
| VB 1 | 91,03 | 99,95 |
| B 1 | 99,41 | 99,99 |

Der erfindungsgemäße Katalysator weist unter den gleichen Reaktionsbedingungen eine signifikant höhere Aktivität bei vergleichbarer oder geringfügig besserer Selektivität auf.

Über die Anwendungsbeispiele 1 und 2 hinausgehend ist der erfindungsgemäße Katalysator für die Hydrierung von Nitrogruppen, die Hydrierung von Iminen, die Hydrierung von Nitrilen, die Hydrierung von C-C-Doppel- und C-C-Dreifachbindungen, die Hydrierung von Carbonylverbindungen, die Hydrierung von CO, CO$_2$ und deren Mischungen, die Hydrierung von Zuckern und die Hydrierung von aromatischen Ringen geeignet.

Er ist erhältlich durch Herstellen einer Mischung aus den Pulvern wenigstens einer Katalysatorlegierung mit einem kleinvolumigen Phasengefüge, das aufgrund einer quantitativen, metallographischen Untersuchung eine spezifische Grenzflächendichte der volumenmäßig größten Phase von größer 0,5 μm$^{-1}$ aufweist, und gegebenenfalls einen oder mehrerer Promotoren enthält, wobei die Katalysatorlegierungen jeweils ein katalytisch aktives Katalysatormetall und wenigstens eine auslaugbare Legierungskomponente enthalten. Durch Zugabe von Befeuchtungsmitteln und/oder Zuschlagstoffen wie Verformungshilfsmitteln, Gleitmitteln, Plastifizierern und/oder Porenbildnern wird eine homogenisierte Mischung hergestellt und zu dem gewünschten Formkörper verformt. Nach Kalzinieren des Formkörpers und Aktivieren der so erhaltenen Katalysatorvorstufe durch teilweises Auslaugen der auslaugbaren Legierungskomponente sowie nach abschließendem Waschen wird der fertige Katalysator erhalten.

**Patentansprüche**

1.    Geformter, aktivierter Metall-Festbettkatalysator mit einem Porenvolumen von 0,05 bis 1 ml/g und einer äußeren, aktivierten Schale, bestehend aus einer versinterten, feinteiligen Katalysatorlegierung sowie gegebenenfalls Promotoren, wobei die Katalysatorlegierung aus der Herstellung der Legierung resultierende metallurgische Phasendomänen aufweist, deren volumenmäßig größte Phase eine spezifische Grenzflächendichte von mehr als $0,5\,\mu m^{-1}$ besitzt.

**2.** Metall-Festbettkatalysator nach Anspruch 1,
**dadurch gekennzeichnet,**
daß es sich bei der Katalysatorlegierung um eine Legierung der Katalysatormetalle Nickel, Kobalt, Kupfer oder Eisen oder Mischungen davon mit einer auslaugbaren Legierungskomponente aus der Gruppe Aluminium, Zink, Silizium oder Mischungen davon, insbesondere Mischungen von Aluminium mit Zink oder Silizium, handelt, wobei Katalysatormetalle und auslaugbare Legierungskomponenten in einem Gewichtsverhältnis von 80 : 20 bis 20 : 80 zueinander stehen.

**3.** Metall-Festbettkatalysator nach Anspruch 1,
**dadurch gekennzeichnet,**
daß als Promotoren die Übergangsmetalle der Gruppen IIIb bis VIIb und VIII und der Gruppe IB des periodischen Systems der Elemente sowie die Seltenerdmetalle in einer Menge von bis zu 20 Gew.-% , bezogen auf das Gesamtgewicht des Katalysators, vorliegen.

**4.** Verwendung des Katalysators nach einem der vorstehenden Ansprüche für die Hydrierung von Nitrogruppen, die Hydrierung von Iminen, die Hydrierung von Nitrilen, die Hydrierung von C-C-Doppel- und C-C-Dreifachbindungen, die Hydrierung von Carbonylverbindungen, die Hydrierung von CO, $CO_2$ und deren Mischungen, die Hydrierung von Zuckern und die Hydrierung von aromatischen Ringen.

**Claims**

**1.** Formed, activated base metal fixed bed catalyst with a pore volume of 0.05 to 1 ml/g and an external, activated shell, consisting of a sintered, fine-particle catalyst alloy and optionally promoters, wherein the catalyst alloy shows metallurgical phase domains resulting from the manufacture of the alloy, of which the largest phase by volume has a specific interfacial density of more than $0.5\mu m^{-1}$.

**2.** Base metal fixed bed catalyst as claimed in claim 1, characterised in that,
the catalyst alloy is an alloy of the catalyst metals nickel, cobalt, copper or iron or mixtures thereof with an extractable alloy component from the group aluminium, zinc, silicon or mixtures thereof, in particular mixtures of aluminium with zinc or silicon, wherein the catalyst metals and extractable alloy components have a weight ratio to each other of 80:20 to 20:80.

**3.** Base metal fixed bed catalyst as claimed in claim 1, characterised in that,
the transitional metals of groups IIIb to VIIb and VIII and of group IB of the periodic system of elements and the rare-earth metals are present in quantities of up to 20 wt.% in relation to the total weight of the catalyst.

**4.** Use of the catalyst as claimed in one of the above claims for the hydrogenation of nitro groups, the hydrogenation imines, the hydrogenation of nitriles, the hydrogenation of C-C double and C-C triple bonds, the hydrogenation of carbonyl compounds, the hydrogenation of CO, $CO_2$ and mixtures thereof, the hydrogenation of sugars and the hydrogenation of aromatic rings.

**Revendications**

**1.** Catalyseur en lit fixe métallique, activé, moulé ayant un volume de pores allant de 0,05 % à 1 ml/g et une coque externe activée consistant en un alliage de catalyseur fritté, finement divisé, ainsi qu'éventuellement des promoteurs, dans lequel l'alliage de catalyseur possède des domaines de phase métallurgiques résultant de la préparation de l'alliage, dont la plus grande phase en ce qui concerne le volume possède une densité spécifique interfaciale de plus de $0,5\,\mu m^{-1}$.

**2.** Catalyseur en lit fixe métallique selon la revendication 1,
caractérisé en ce qu'
il s'agit, en ce qui concerne l'alliage de catalyseur, d'un alliage des métaux de catalyseur nickel, cobalt, cuivre ou fer ou des mélanges de ceux-ci, avec un composant d'alliage éliminable par lessivage choisi dans le groupe de l'aluminium, du zinc, du silicium ou des mélanges de ceux-ci, en particulier des mélanges d'aluminium avec le zinc ou le silicium, dans lequel les métaux de catalyseur et les composants d'alliage éliminables par lessivage, se situent l'un par rapport à l'autre dans un rapport pondéral allant de 80 : 20 à 20 : 80.

3. Catalyseur en lit fixe métallique selon la revendication 1,
caractérisé en ce que
comme promoteurs on introduit les métaux de transition des groupes IIIb à VIIb et VIII et du groupe Ib du système périodique des éléments ainsi que les métaux des terres rares en une quantité allant jusqu'à 20 % en poids, rapporté au poids total du catalyseur.

4. Utilisation du catalyseur selon l'une des revendications précédentes pour l'hydrogénation de groupes nitro, l'hydrogénation d'imines, l'hydrogénation de nitriles, l'hydrogénation de double liaisons C-C et de triple liaisons C-C, l'hydrogénation de composés carbonylés, l'hydrogénation de CO, de $CO_2$ et de leurs mélanges, l'hydrogénation de sucres et l'hydrogénation de cycles aromatiques.